# EUROPEAN PATENT APPLICATION

(11) **EP 2 799 880 A1**
(43) Date of publication of application: **05.11.2014**
(21) Application number: 12862839.3
(22) Date of filing: 26.12.2012
(51) Int. Cl.: G01N 33/86, G01N 35/08, G01N 33/49

(54) **APPARATUS AND METHOD FOR MEASURING HEMOSTATIC FUNCTION USING BLOOD MOBILITY**

(30) Priority: 27.12.2011 KR 20110143242
(71) Applicant: Korea University Research and Business Foundation, Seoul 136-713 (KR)
(72) Inventor: LIM, Chae Seung, Anyang-si Gyeonggi-do 431-070 (KR); SHIN, Se Hyun, Seoul 137-060 (KR); KIM, Kyoeng A, Seoul 122-876 (KR)
(74) Representative: Vossius & Partner
(86) International application number: PCT/KR2012/011426
(87) International publication number: WO 2013/100536

(57) **Abstract**

The present invention relates to an apparatus and a method for measuring a hemostatic function using blood mobility. The present invention can comprise a measurement kit; and one or more capillaries provided at the measurement kit in which a blood inlet is formed at one end and an opening communicating with the outside is formed at the other end. In addition, recesses inwardly recessed can be formed longitudinally on at least one lateral side of the capillaries. According to the present invention, it is possible to rapidly measure the hemostatic function of blood and to examine multiple samples at one time by measuring the mobility of a very small amount of blood moving the capillaries.

## Description

### [Technical Field]

The present invention relates to a device and method for measuring a hemostatic function using a blood migration ratio, and more specifically, to a device and method for measuring a hemostatic function using a blood migration ratio in which platelet abnormality may be easily determined by measuring a migration distance of blood inside a reagent-treated tube and measuring a hemostatic function.

### [Background Art]

A method of measuring a hemostatic function of blood is widely used for screening congenital platelet dysfunctions or a preoperative screening test, and particularly, is an important test for screening hemorrhagic diseases due to congenital or acquired platelet dysfunctions in hemorrhagic diseases having no numerical platelet disorders.

Recently, the method is being widely used for testing increased bleeding tendency and drug resistance due to antiplatelet drugs used to treat and prevent cardiovascular diseases.

A currently used blood hemostatic function measurement method is a bleeding time (BT) test that was developed about 100 years ago and is still frequently used as a platelet function screening test. The bleeding time test is performed by inducing bleeding mainly using a lancet and then measuring a time until bleeding stops. Detailed methods include a Duke method, an Ivy method, a Template method, and the like.

However, these methods have problems in that it is difficult to standardize bleeding time measurement, clinical usefulness is low, and an invasive method is required. Therefore, a simple and objective measurement method is required.

In addition, PFA-100, which is currently used in hospitals and uses an automatic blood hemostatic function test method, is a test device capable of measuring platelet functions using a pressure change until hemostasis is completed when blood moves in vitro. This device provides an in-vivo environment similar to when an in-vitro bleeding time is measured, but it takes a long measurement time of about 100 to 150 seconds, and only one blood sample may be tested at a time.

### [Disclosure]

### [Technical Problem]

In view of the above-described problems in the related art, the present invention provides a device and method for measuring a hemostatic function using a blood migration ratio in which a measurement time is short when a blood hemostatic function is measured and a plurality of kits are included such that a plurality of blood samples may be simultaneously measured.

### [Technical Solution]

According to an aspect of the present invention, the present invention includes a measurement kit; and at least one capillary provided in the measurement kit and having one end in which an inlet into which blood is injected is formed and the other end in which an aperture communicating with the outside is formed.

A recessed portion inwardly recessed from at least one side along a longitudinal direction of the capillary may be formed.

The recessed portion may be symmetrically formed in both sides along a longitudinal direction of the capillary.

The recessed portion may be formed in both sides in the form of a zigzag along a longitudinal direction of the capillary.

At least one resistance portion having a small flow area may be formed in the capillary.

Graduations may be formed to display a blood migration distance in the capillary.

The device may further include an abnormal blood detecting sensor disposed in a position outside of a normal blood migration distance and configured to detect the blood.

The device may further include a controller configured to receive a signal detected by the abnormal blood detecting sensor and determine whether a hemostatic function of blood is normal.

The measurement kit may be disposable.

Any reagent of collagen and epinephrine, and collagen and ADP may be mixed with and injected into the blood.

The collagen may be 1.84 mg/ml or more, and the ADP may be 37.5 mg/ml or more.

According to another aspect of the present invention, the present invention includes injecting blood into an inlet formed in one end of a capillary; measuring a migration distance or a migration completion time of the blood injected into the inlet; and when the migration distance or the migration completion time of the blood is within a normal range, determining a hemostatic function as normal, and otherwise, determining a hemostatic function as abnormal.

The method may further include detecting blood outside of a normal migration distance; and determining whether a hemostatic function of blood is normal by receiving a detected signal of the blood.

### [Advantageous Effects]

According to the present invention, a blood migration ratio of a small amount of blood migrating through a capillary is measured using a disposable measurement kit. As a result, it is possible to rapidly measure a hemostatic function of blood and perform a test on a plurality of samples at a time.

### [Description of Drawings]

FIG. 1 is a plan view illustrating a hemostatic function measurement device using a blood migration ratio according to an embodiment of the present invention.
FIG. 2 is a plan view illustrating a hemostatic function measurement device using a blood migration ratio according to another embodiment of the present invention.
FIG. 3 is a diagram illustrating a comparison result obtained by measuring a hemostatic function of blood according to an embodiment of the present invention.
FIG. 4 shows pictures of the results obtained by measuring changes in blood resulting from a concentration difference of collagen and ADP according to an embodiment of the present invention.
FIG. 5 shows pictures of the results obtained by measuring changes in blood resulting from a concentration difference of collagen and ADP according to an embodiment of the present invention.
FIG. 6 is a diagram illustrating a migration process of blood and a comparison result of a blood migration ratio according to a concentration difference of collagen and ADP of an embodiment of the present invention.
FIG. 7 is a graph showing the result obtained by comparing a decrease in a blood migration ratio according to a reaction time and a reaction temperature after ADP is added.
FIG. 8 shows pictures of specimens having a decreased blood migration ratio according to a reaction time after ADP is added.
FIG. 9 shows pictures of the results obtained by measuring a change in a blood migration distance according to a reaction time of an embodiment of the present invention.
FIG. 10 shows pictures of the results obtained by measuring a blood migration distance change according to a reaction time of another embodiment of the present invention.
FIG. 11 illustrates graphs showing the results obtained by measuring a PFA-100 platelet function measurement device and a blood migration ratio after a platelet antiaggregant is injected.
FIG. 12 illustrates graphs showing the results measured by flow cytometry after a platelet antiaggregant is injected.

### [Modes of the Invention]

Hereinafter, a hemostatic function measurement device using a blood migration ratio according to an embodiment of the present invention will be described in detail with reference to the accompanying drawings.

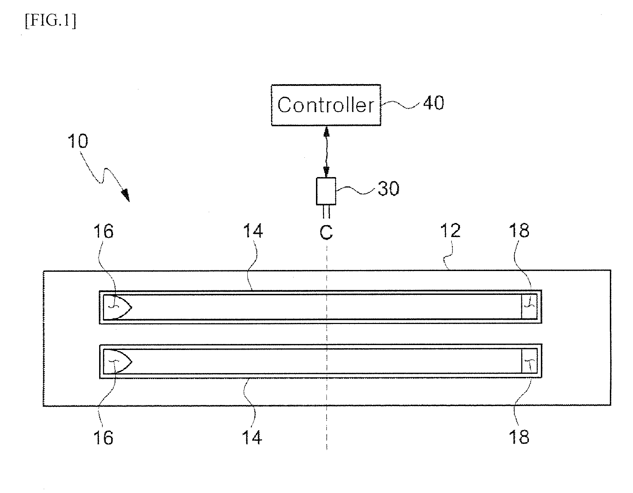

FIG. 1 is a plan view illustrating a hemostatic function measurement device using a blood migration ratio according to an embodiment of the present invention.

As illustrated, a hemostatic function measurement device using a blood migration ratio 10 according to an embodiment of the present invention may include a measurement kit 12 and at least one capillary 14 provided in the measurement kit 12 and having one end in which an inlet 16 into which blood is injected is formed and the other end in which an aperture 18 communicating with the outside is formed.

The measurement kit 12 is a small kit for measuring a hemostatic function according to blood coagulation and may be manufactured to be disposable. When the measurement kit 12 is manufactured to be disposable in this manner, it is possible to easily measure blood coagulation using a miniaturized instrument and also measure a small amount of blood coagulation. The measurement kit 12 may be made of any material of, for example, a laser-processable polymer, an injection molding polymer, and a ceramic. Needless to say, the material of the measurement kit 12 is not limited to the above materials.

At least one capillary 14 may be disposed in parallel in the measurement kit 12. While two capillaries 14 are disposed in parallel in FIG. 1, the present invention is not limited thereto, but three or more capillaries 14 may be disposed in parallel. Therefore, even when a plurality of blood samples are tested, it is possible to simultaneously test whether the hemostatic function is abnormal through the plurality of capillaries 14 using the single measurement kit 12.

The capillary 14 needs to be manufactured to have a longitudinal direction and a width which can cause a capillary phenomenon. In addition, the capillary 14 may be manufactured to have a cross section of a circular shape or a polygonal shape such as a triangular shape or a rectangular shape.

In the present embodiment, the capillary 14 may be manufactured in various sizes depending on usage purposes. However, in general, it is preferable to have a longitudinal direction of 60 mm, a width of 2 mm, and a depth of 0.5 mm. Also, the capillary 14 may be optically transparently manufactured such that observation is possible from the outside.

In addition, a test may be performed in an upright state of the capillary 14 in which the inlet 16 is lowered or may also be performed in a state in which the capillary 14 is located on a bottom surface.

Also, the inlet 16 for injecting blood is formed in one end of the capillary 14. The inlet 16 may have, for example, a semicircular shape with respect to a migration direction of blood as in FIG. 1, or may be formed in various shapes such as a circular shape, a triangular shape, and a rectangular shape.

In addition, the aperture 18 for communicating between an inside and an outside of the capillary 14 is formed in the other end of the capillary 14. The aperture 18 may be formed in, for example, a rectangular shape as in FIG. 1, or may be formed in various shapes such as a circular shape and a triangular shape. When the aperture 18 is formed in this manner, blood injected into one end may migrate through the capillary 14.

Meanwhile, the hemostatic function measurement device 10 according to the embodiment of the present invention further includes an abnormal blood detecting sensor 30 that is disposed in a position outside of a normal blood migration distance. The abnormal blood detecting sensor 30 is configured to detect when blood injected into the inlet 16 deviates from a normal migration distance. For example, in FIG. 1, if it is assumed that a hemostatic function of blood is normal when blood migrates within a center line (C), the abnormal blood detecting sensor 30 is disposed to be placed along an extension line of the center line (C). Needless to say, the abnormal blood detecting sensor 30 may be disposed in an area within an expected error range with respect to the normal blood migration distance.

When the abnormal blood detecting sensor 30 detects migration of blood, a detection signal is transmitted to a controller 40. When the signal is transmitted from the abnormal blood detecting sensor 30, the controller 40 determines that the hemostatic function of blood is abnormal, and displays abnormality through a display unit (not illustrated) and the like. In addition, it may be configured such that a sound indicating an abnormal hemostatic function is output to the outside through a sound signal output unit provided in the controller 40.

While the abnormal blood detecting sensor 30 and the controller 40 have been exemplified above as a device for determining the hemostatic function measurement device 10, the present invention is not limited thereto. Graduations are displayed on the capillary 14 to check a normal migration distance range of blood.

In addition, in the present embodiment, any reagent of collagen and epinephrine, and collagen and ADP which are coagulants may be mixed with and injected into blood.

Hereinafter, a hemostatic function measurement device using a blood migration ratio according to another embodiment of the present invention will be described in detail with reference to the accompanying drawings.

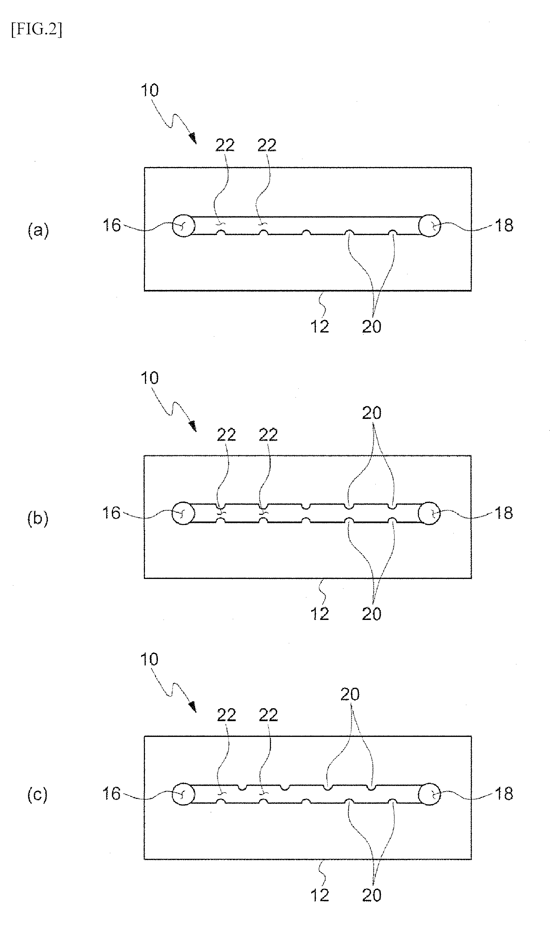

FIG. 2 is a plan view illustrating a hemostatic function measurement device using a blood migration ratio according to another embodiment of the present invention.

As illustrated, unlike the above-described embodiment, in the present embodiment, a recessed portion 20 inwardly recessed along a longitudinal direction of the capillary 14 may be formed. Here, the recessed portion 20 may be formed in various positions. As the recessed portion 20, as illustrated in FIG. 2A, for example, the recessed portion 20 inwardly recessed from one side along a longitudinal direction of the capillary 14 may be formed. The plurality of recessed portions 20 may be formed at predetermined intervals in a longitudinal direction direction of the capillary 14. When the recessed portions 20 are formed in this way, an analysis ability of the blood migration distance increases.

Meanwhile, as illustrated in FIG. 2B, the recessed portions 20 may be symmetrically formed in both sides with respect to the longitudinal direction direction of the capillary 14.

Also, as illustrated in FIG. 2C, the recessed portion 20 may be formed in both sides in the form of a zigzag with respect to the longitudinal direction direction of the capillary 14. In this manner, the recessed portion 20 may be formed in various positions according to blood properties, experiment conditions, and the like.

Also, a shape of the recessed portion 20 described above is not limited to the shape illustrated in FIG. 2. That is, while FIG. 2 illustrates that the recessed portion 20 is formed to be round and blood flows smoothly over the recessed portion 20, the present invention is not limited thereto, but the recessed portion 20 may have various shapes such as a triangular shape and a rectangular shape.

Meanwhile, as a result, the recessed portion 20 described above forms a flow resistance portion 22 having a small flow area in a flow path of blood. That is, the flow resistance portion 22 is a portion having a decreased flow area due to the recessed portion 20 inside the capillary 14, and at least one flow resistance portion may be formed across the entire capillary 14. When the flow resistance portion 22 is formed in the capillary 14 in this manner, blood has increased flow resistance and an increased resolution.

According to the hemostatic function measurement device 10 described in the above-described embodiment, a hemostatic function of blood is determined as normal or abnormal based on a migration distance and a migration completion time of blood.

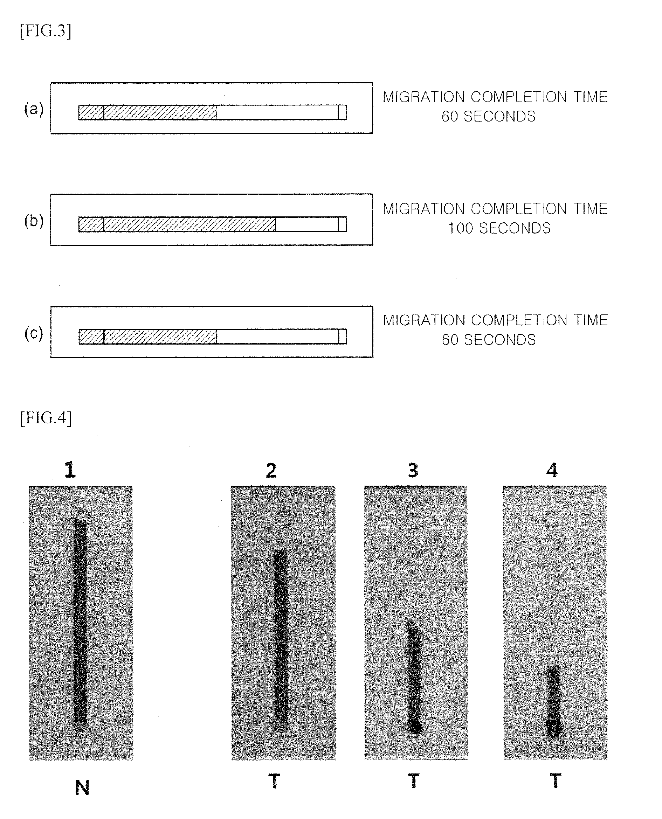

FIG. 3 illustrates a comparison result obtained by measuring a hemostatic function of blood according to an embodiment of the present invention.

As illustrated in FIG. 3A, when the reagent such as collagen and epinephrine, and collagen and ADP is added to drawn blood, a blood migration distance of a normal subject is measured. As illustrated, the blood migration distance of the normal subject is, for example, 10 mm until complete coagulation, and a migration completion time is 60 seconds.

Next, FIG. 3B illustrates a blood migration distance having an abnormal hemostatic function. As illustrated, when blood has an abnormal hemostatic function, a migration distance until complete coagulation is 14 mm and a migration completion time is 100 seconds. Therefore, a tester determines that blood in FIG. 3B has an abnormal hemostatic function, and may begin treatment corresponding thereto.

Next, the tester determines that blood in FIG. 3C has a normal hemostatic function since it has the same migration distance and migration completion time as the blood in FIG. 3A which is determined as normal.

### Example 1

FIG. 4 shows pictures of the results obtained by measuring changes in the blood resulting from a concentration difference of collagen and ADP according to an embodiment of the present invention.

As illustrated in FIG. 4, "T" indicates blood into which a reagent of collagen and ADP is injected and "N" indicates blood into which no reagent is injected.

Four samples were prepared and concentrations of reagents of collagen and ADP injected into each sample are as follows.
(1) Sample 1: collagen 0 mg/ml and ADP 0 mg/ml
(2) Sample 2: collagen 1.84 mg/ml and ADP 25 mg/ml
(3) Sample 3: collagen 1.84 mg/ml and ADP 30 mg/ml
(4) Sample 4: collagen 1.84 mg/ml and ADP 37.5 mg/ml

In this way, changes in the blood are measured by changing the concentration of the reagent of collagen and ADP in each sample. As a result, it can be seen that blood into which 37.5 mg/ml of ADP reagent is injected has a relatively short blood migration distance.

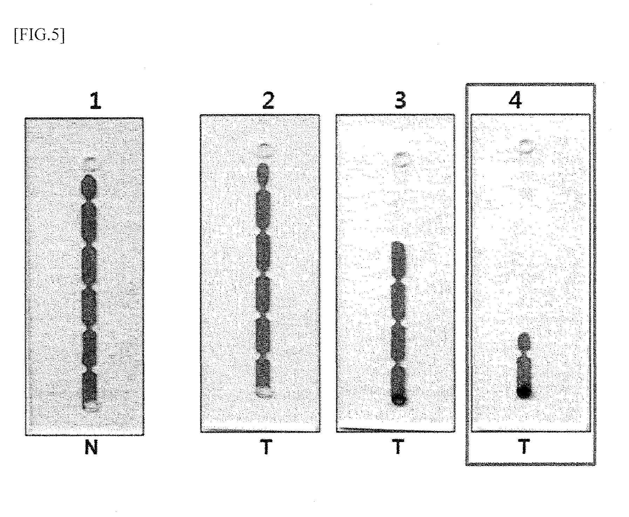

FIG. 5 shows pictures of the results obtained by measuring changes in blood resulting from a concentration difference of collagen and ADP according to an embodiment of another chamber microstructure of the present invention.

As illustrated, similarly, in FIG. 5, "T" indicates blood into which collagen and ADP are injected and "N" indicates blood into which no reagent is injected.

Four samples were prepared and concentrations of reagents of collagen and ADP injected into each sample are as follows.
(1) Sample 1: no reagent is injected
(2) Sample 2: collagen 1.84 mg/ml and ADP 25 mg/ml
(3) Sample 3 collagen 1.84 mg/ml and ADP 31.25 mg/ml
(4) Sample 4: collagen 1.84 mg/ml and ADP 37.5 mg/ml

In this way, changes in the blood are measured by changing the concentration of collagen and ADP in each sample. As a result, it can be seen that the blood into which 1.84 mg/ml of collagen and 37.5 mg/ml of ADP are injected has a relatively short blood migration distance.

Based on the above-described examples, it was determined that the most appropriate injection concentrations of collagen and ADP are 1.84 mg/ml and 37.5 mg/ml.

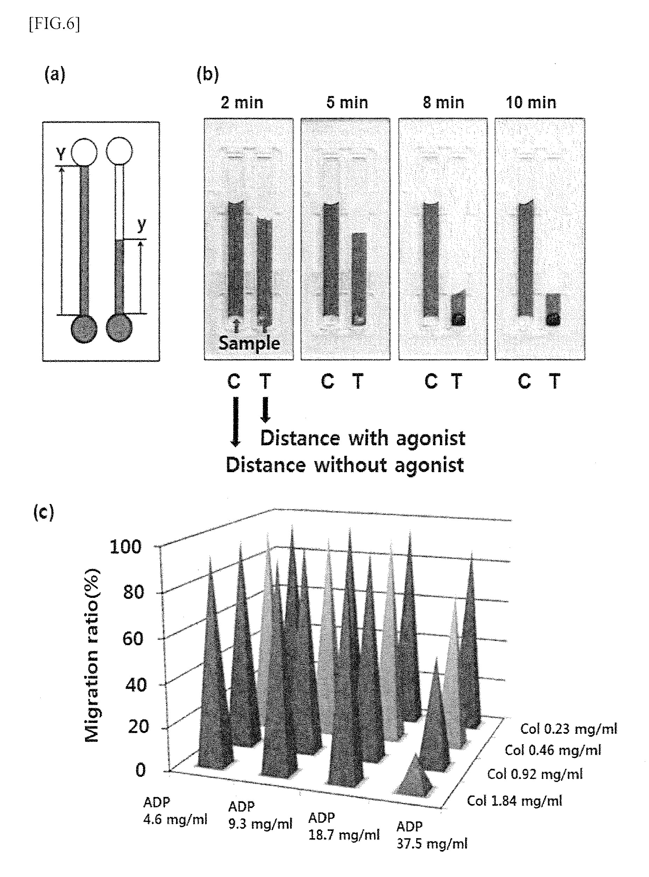

FIG. 6A illustrates a method of calculating a blood migration ratio. When no ADP serving as a platelet aggregation agonist is injected, blood may migrate to the end. However, when ADP is injected, blood coagulates midway and migration stops. At this time, a ratio with respect to each migration distance of blood is represented by y/Y* 100%.

FIG. 6B shows pictures of the results obtained by observing an actual blood migration distance by time. As time elapses after the reagent is injected, a blood migration ratio decreases from 95% to about 30%.

FIG. 6C illustrates an experiment for determining optimum injection concentrations of ADP and collagen. Here, when 1.84 mg/ml of collagen and 37.5 mg/ml of ADP are used, an optimum coagulation phenomenon occurs and a blood migration ratio decreases.

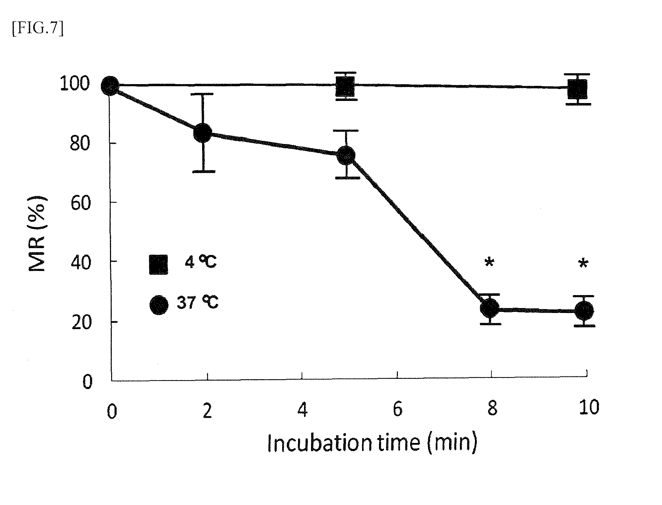

FIG. 7 is a graph showing the result obtained by comparing a decrease in a blood migration ratio according to a reaction time and a reaction temperature after ADP serving as a platelet aggregation agonist is added. According to the graph, a migration ratio decreases due to maximum coagulation between 8 to 10 minutes at 37 °C.

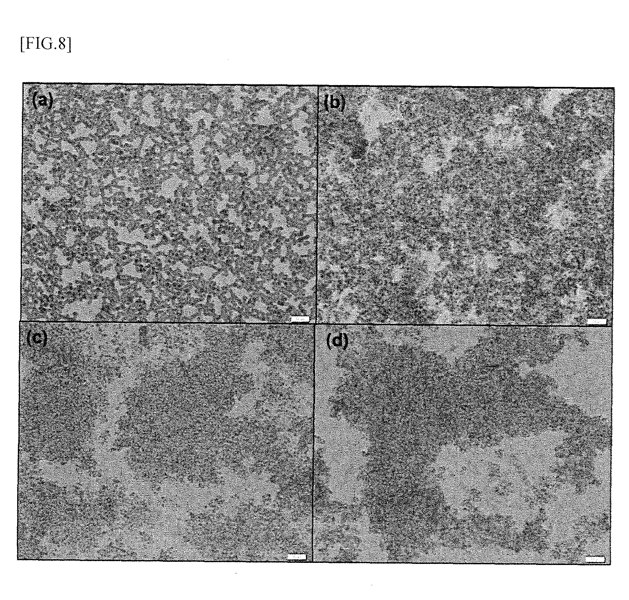

FIG. 8 shows pictures of specimens having a decreased blood migration ratio according to a reaction time after ADP serving as a platelet aggregation agonist is added. Here, it may be observed that coagulation largely increases at time (d).

### Example 2

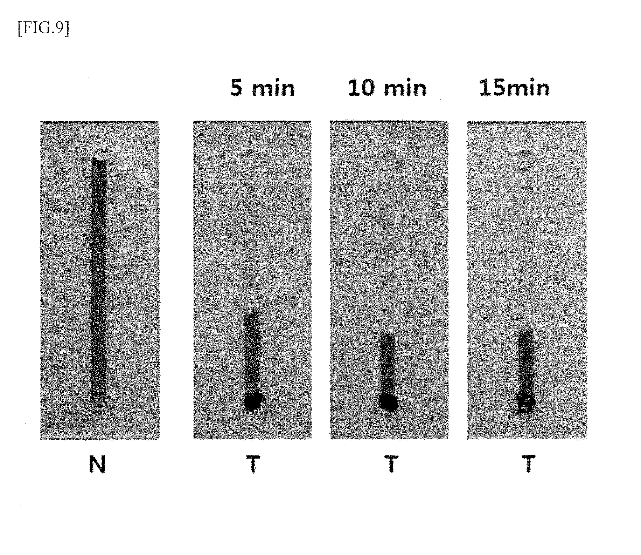

FIG. 9 shows pictures of the results obtained by measuring changes in blood according to a reaction time of another embodiment of the present invention.

As illustrated, "T" indicates a sample in which optimum concentrations, 1.84 mg/ml of collagen and 37.5 mg/ml of ADP, derived from Example 1 are treated. "N" indicates blood into which no reagent is injected.

After blood was injected, changes in each blood sample were measured at intervals of 5 minutes, 10 minutes, and 15 minutes. Similar to FIG. 6, it can be seen that, as time elapsed, the blood migration distance increased more when 10 minutes had elapsed than when 5 minutes had elapsed, and the migration distance decreased again when 15 minutes had elapsed. Here, the decrease in the migration distance may be supposed to be caused by blood migration stopping since bleeding stops due to coagulability of platelets.

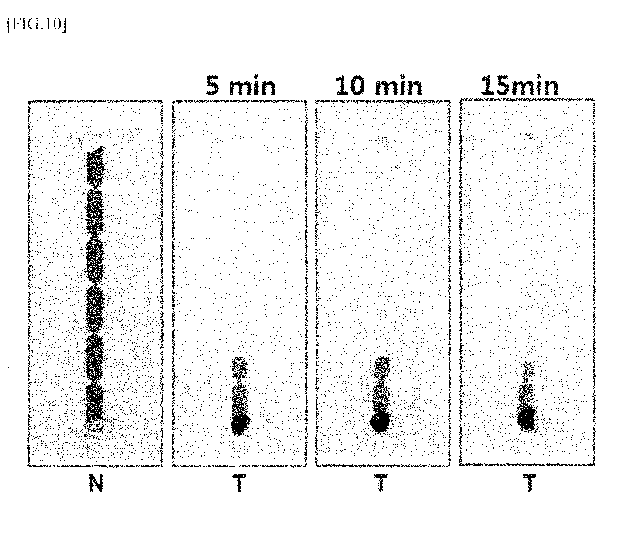

FIG. 10 shows pictures of the results obtained by measuring a blood migration distance change according to a reaction time of another embodiment of the present invention.

As illustrated, similar to FIG. 9, changes in each blood sample are measured at intervals of 5 minutes, 10 minutes, and 15 minutes after blood is injected. It can be seen that the migration distance of the blood sample decreased as time elapses.

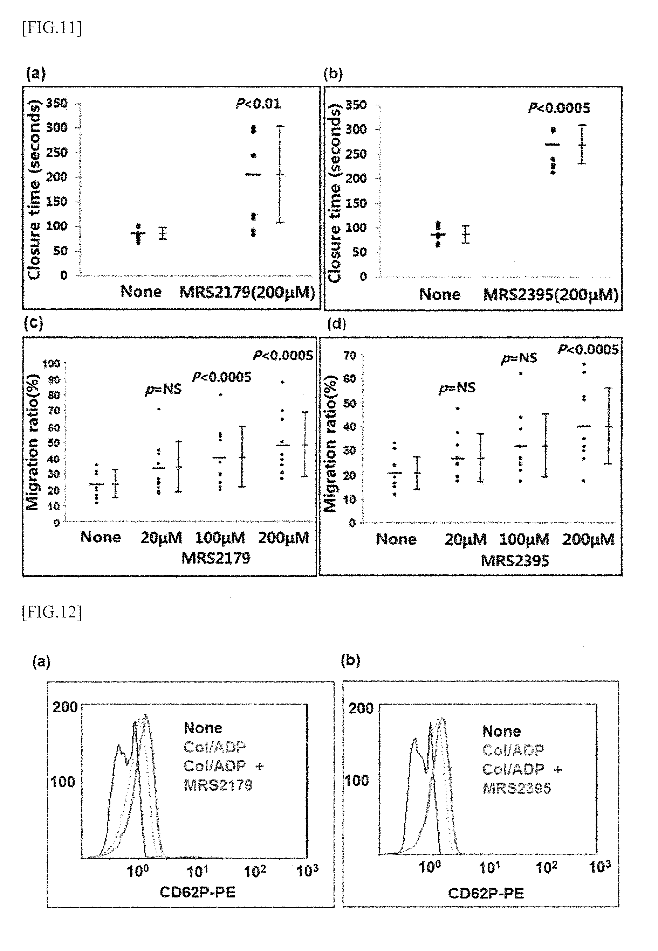

Meanwhile, FIG. 11 illustrates graphs showing the results obtained by measuring a PFA-100 platelet function measurement device and a blood migration ratio after a platelet antiaggregant is injected. Specifically, after a platelet antiaggregant called MRS2179 or MRS2395 is artificially injected, an abnormal condition and a normal condition are prepared, and the PFA-100 platelet function measurement device and a blood migration ratio are measured. Here, as a concentration of the platelet antiaggregant increases, a closure time of PFA-100 and a blood migration ratio are changed to abnormal.

FIG. 12 illustrates graphs showing the results measured by flow cytometry after a platelet antiaggregant is injected. Specifically, after a platelet antiaggregant called MRS2179 or MRS2395 is artificially injected, an abnormal condition and a normal condition are prepared and measurement is performed using flow cytometry. It may be observed that P-selectin (cd62) maximally increases in the normal condition. However, in the abnormal condition, it may be observed that expression of platelets decreases to a medium level (blue part).

The scope of the present invention is not limited to the above-described examples, but defined by the appended claims. It is apparent that those skilled in the art may perform various modifications and alternations within the scope of the appended claims.

## Claims

1. A hemostatic function measurement device using a blood migration ratio, comprising:
a measurement kit; and
at least one capillary provided in the measurement kit and having one end in which an inlet into which blood is injected is formed and the other end in which an aperture communicating with the outside is formed.

2. The device of claim 1,
wherein a recessed portion inwardly recessed from at least one side along a longitudinal direction of the capillary is formed.

3. The device of claim 1,
wherein a recessed portion is symmetrically formed in both sides along a longitudinal direction of the capillary.

4. The device of claim 1,
wherein a recessed portion is formed in both sides in the form of a zigzag along a longitudinal direction of the capillary.

5. The device of claim 1,
wherein at least one resistance portion having a small flow area is formed in the capillary.

6. The device of claim 1,
wherein graduations are formed to display a blood migration distance in the capillary.

7. The device of claim 1, further comprising
an abnormal blood detecting sensor disposed in a position outside of a normal migration distance of the blood and configured to detect the blood.

8. The device of claim 7, further comprising
a controller configured to receive a signal detected by the abnormal blood detecting sensor and determine whether a hemostatic function of blood is normal.

9. The device of claim 1,
wherein the measurement kit is disposable.

10. The device of claim 1,
wherein any reagent of collagen and epinephrine, and collagen and ADP is mixed with and injected into the blood.

11. The device of claim 10,
wherein the collagen is 1.84 mg/ml or more, and the ADP is 37.5 mg/ml or more.

12. A method of measuring a hemostatic function using a blood migration ratio, the method comprising:
injecting blood into an inlet formed in one end of a capillary;
measuring a migration distance or a migration completion time of the blood injected into the inlet; and
when the migration distance or the migration completion time of the blood is within a normal range, determining a hemostatic function as normal, and otherwise, determining a hemostatic function as abnormal.

13. The method of claim 12, further comprising
detecting blood outside of a normal migration distance; and
determining whether a hemostatic function of blood is normal by receiving a detected signal of the blood.
